# EUROPEAN PATENT APPLICATION

(11) **EP 1 393 710 A1**
(43) Date of publication of application: **03.03.2004**
(21) Application number: 02255842.3
(22) Date of filing: 21.08.2002
(51) Int. Cl.: A61K 7/16

(54) **A method of applying an oral composition**

(71) Applicant: THE PROCTER & GAMBLE COMPANY, Cincinnati, Ohio 45202 (US)
(72) Inventor: Moneuze, Gaelle, Windlesham, Surrey GU20 6QD (GB); Strand, Ross, Warfield, Bracknell, Berkshire RG42 3RZ (GB); White, Christopher David, Richmond, Surrey TW9 3EB (GB); Williams, Michael Kevin, Wokingham, Berkshire RG40 2HU (GB)
(74) Representative: Clemo, Nicholas Graham

(57) **Abstract**

The present invention relates to a method of applying oral care benefit agent to the oral tissues. More specifically, a method for treating an oral cavity is provided comprising the application of an aqueous composition comprising oral care benefit agents to a large proportion of the oral cavity, application occurring as part of the daily oral care routine shortly before retiring, and the composition remaining in contact with the oral tissues while sleeping. The method and aqueous compositions of the invention provide overnight application and delivery of oral care benefit agents with improved ease of use and consumer aesthetics

## Description

### FIELD

The present invention relates to a method of applying oral care benefit agent to the oral tissues. More specifically, the invention relates to a method of application of an oral composition such that the oral composition remains in contact with the oral tissues overnight during rest or slumber.

### BACKGROUND

The benefits of maintaining oral hygiene are well understood. Consumers understand the benefits of daily oral treatments such as brushing teeth and the use of mouth rinses. These benefits include the reduction of caries, plaque, and gingivitis; treating hypersensitivity; freshening breath; whitening teeth and removing stains; remineralising teeth and the like. An increasing consumer requirement is the need to maintain their teeth for life. Consumers relate healthy oral tissues and "fresh breath" with a healthy body and lifestyle. A wide variety of oral care products have been developed to aid in the short-term maintenance of good oral hygiene. These products deliver various oral care benefit agents to the soft and hard tissues of the oral cavity in such a way that, in general, they are intended for application by the consumer themselves during part of their daily routine, and/or are administered by oral hygiene specialists in the course of administering treatment.

The most frequently used oral care treatments used in the western world are those treatments that are administered by the consumer themselves once or twice a day as part of the daily routine. Examples of such treatments include dentifrices containing for example anti-bacterial plaque actives and/or anti-caries actives and mouth rinses containing anti-bacterial actives and/or breath freshening actives. The applicant is aware that it is a need of the consumer to have 24-hour maintenance of oral health. Whilst some of these treatments claim extended or prolonged therapeutic benefit following the initial treatment, they do not typically meet the needs of the consumer in providing substantial long lasting therapeutic, prophylactic and/or cosmetic treatment benefits. As a result, the only way to achieve sustained active release has been to periodically reapply the product, or to use special delivery mechanisms such as a dental tray. Also, despite the common acceptance and use of extended daily oral regimens such as brushing, rinsing and flossing, unsatisfactory morning mouth feel and malodour are still a consumer concern.

The existence of "morning breath" and the conditions associated with it indicate that even the application of existing daily oral care regimens prior to retiring in the evening have little effect on the degeneration of oral health overnight. During slumber, reduced salivary flow and excessive growth of anaerobic bacteria result in conditions well suited to the degeneration of the oral tissues and the development of oral malodour and gingivitis. Coupled with reduced pH control in the oral cavity, these conditions are optimal for the development of oral conditions such as caries, gingivitis and plaque formation. Such processes are ongoing, and though they may be reduced or modified by existing treatments, they can only be effectively treated, either prophylactically or therapeutically, by continuous attention, which is impractical, or by the use of long lasting treatments.

Several attempts have been made to provide products having enhanced substantivity and prolonged oral tissue contact times with the objective of increasing the exposure of oral care benefit agents to the oral tissues. These attempts include the use of water-soluble and -insoluble film forming polymers to deliver various actives to the oral tissues, specifically the hard tissues. US 5,462,728 teaches a water insoluble bioadhesive copolymer matrix containing a therapeutic agent to be applied to the oral cavity. Such water insoluble vehicles are designed to precipitate the polymeric carrier and active agent contained therein upon application to the oral cavity, and are designed for treatment of small areas of the oral cavity. Also, such polymeric films require removal by mechanical means such as brushing. This may also result in a palpable hard coating being formed on the oral tissues that is unpleasant to the consumer. US 5,462,728 further discloses a method of applying the oral composition to the oral cavity resulting in the in situ formation of an adhering, water insoluble film that remains active for a period of hours.

US 5,425,953 teaches the application of compositions comprising cellulosic polymers with high levels of ethyl alcohol and carbamide peroxide. Following application, the solvent evaporates, leaving a polymeric film on the teeth delivering the contained carbamide peroxide. The compositions therein are disclosed as being for intermittent or acute application in the treatment of oral conditions. These compositions contain levels of monohydric alcohols above 50% and may cause undesired consumer reactions to palpable layers on the oral tissues. Furthermore, they may require mechanical means of removal following application.

US 5,438,076 teaches the use of acrylic polymers to deliver pharmacological agents to the oral cavity. These compositions are designed for application to afflicted areas of the oral cavity, not the oral cavity as a whole, and may result in palpable film formation that some consumers find unpleasant.

Another method of prolonged delivery of oral care benefit agents is described in WO02/34221. This document discloses the application of oral care compositions comprising a silicone resin, a silicone gum and a silicone fluid and an oral care benefit agent. The oral care compositions disclosed by this document form a substantive film on the surface of the teeth or gums and may be "broadly applied to the whole cavity". Due to the composition's substantivity it will remain on the oral tissues for up to 8 hours and requires removal by mechanical means such as brushing or rinsing. Whilst the compositions of WO02/34221 are excellent for providing long-term delivery of oral care benefits, it has been found that some consumers prefer not to have palpable silicone residues on the tissues of the oral cavity the following morning.

US 5,631,000 teaches the whitening of teeth with an aqueous gel that is exposed to the oral tissues by being placed in a dental tray that is then worn in the oral cavity. The dental tray is usually worn at night, but may be worn during the day. However, dental trays are uncomfortable to wear. Application of oral care overnight without the requirement of a dental tray is advantageous due to the ease of application to the oral cavity, and the good aesthetic experience of the consumer.

It is desirable to have a method that delivers an oral care benefit agent to a consumer whilst sleeping without the requirement of further application or intervention following the initial application. Overnight delivery of oral care would be a suitable remedy to combat the conditions in the oral cavity that develop whilst asleep. Overnight delivery is also advantageous as it is easily incorporated into the every day oral regimen of the consumer without excessive requirement for specialist equipment or knowledge. Furthermore, it would be desirable to have a method of oral care that is applied immediately prior to retiring, and immediately after the application of an existing oral care regimen. Further still, it is desirable that the oral care product has a pleasant mouth feel acceptable for long term use in the oral cavity. Acceptable mouth feel is advantageous as it encourages regular consumer usage. Long-term mouth feel is recognised as a balancing act between substantivity, adherence and viscosity. Desirable products require sufficient substantivity and viscosity to enable application to the oral cavity, to adhere to the oral tissues and to release the contained oral care benefit agents over an extended period of time. However, the viscosity should not be so high that the consumer can feel globular portions of the newly applied product that have not spread well over the oral tissues upon application. It is desirable to have a gel for use in the present invention that enables easy application to the oral cavity, thin layer formation over the oral tissues and even spread into periodontal pockets and fissures

It is a consumer need to awake with a "fresh" mouth feel in the morning, but without oral care products palpably maintained on the oral tissues. Therefore, there is a need for oral care products that satisfactorily deliver oral care benefit agents to the oral tissues overnight, but dissolve within the oral cavity such that, once the consumer awakes, he or she does not feel the presence of the applied product, and the product does not require mechanical means of removal such as brushing or rinsing.

Based on the foregoing, there is need for a method of oral care benefit agent delivery based upon the overnight application of a gel with a rheological profile that results in overnight release of oral care benefit agents onto the oral tissues without palpable residue formation or requirement of removal. Furthermore, there is need for a method of overnight application that covers a large proportion of the oral cavity, is relatively easy and forms part of the daily routine.

### SUMMARY

A method for treating the oral cavity is provided comprising the application of an aqueous gel to the oral cavity such that at least 75% of the gingival margin or at least 75% of the buccal portion of the hard tissue is coated with the gel, application occurring as part of the daily oral care routine shortly before retiring, and the gel remaining in contact with the oral tissues while sleeping, the gel comprising;
a) an oral care benefit agent;
b) a thickener;
c) less than 5% abrasive;
d) less than 18% C₁-C₆ monohydric alcohols;
e) less than 10% silicone; and
the gel further having a viscosity of greater than about 15 Pa.s at a shear rate of 0.1 s⁻¹ and from about 0.1 Pa.s to about 300 Pa.s. at a shear rate of 1 s⁻¹.

The method and aqueous gels provided herein provide means for overnight application and delivery of an aqueous gel comprising oral care benefit agents with improved ease of use and consumer aesthetics.

Herein, "buccal portion" means those surfaces of oral tissues closest to the cheeks and inner surfaces of the lips.

Herein, "thickener" means any material that when added to a solvent or carrier results in the viscosity of the solvent or carrier increasing.

Herein, "abrasive" means any particulate material with polishing or abrasive characteristics that is substantially insoluble in water and has a diameter of from about 1 µm to about 100 µm.

The invention further relates to aqueous gels for use according to the method above comprising;
a) from about 0.1% to about 1.5% by weight of an anti-microbial;
b) from about 1% to about 15% thickener;
c) less than 5% abrasive;
d) less than 18% C₁-C₆ monohydric alcohols;
e) less than 10% silicone; and the aqueous gel being characterised in that it has a viscosity of greater than about 15 Pa.s at a shear rate of 0.1 s⁻¹ and from about 0.1 Pa.s to about 300 Pa.s. at a shear rate of 1 s⁻¹.

The invention further relates to a kit for treatment of the oral cavity comprising an oral care gel, an applicator and method of use instructions directed towards the present invention.

These and other features, aspects, and advantages of the present invention will become evident to those skilled in the art from a reading of the present disclosure.

### DETAILED DESCRIPTION

While the specification concludes with claims that particularly point out and distinctly claim the invention, it is believed the present invention will be better understood from the following description.

All cited references are incorporated herein by reference in their entireties. Citation of any reference is not an admission regarding any determination as to its availability as prior art to the claimed invention.

All percentages are by weight of total composition unless specifically stated otherwise and all measurements are made at 20°C, unless otherwise stated. All ratios are weight ratios unless specifically stated otherwise.

Viscosity of the gel as used herein unless otherwise stated is measured using a Carri-med CSL² rheometer with a gap of 500µm and continuous linear ramps of shear rates from 0.1 to 1 s⁻¹ and 1 to 900 s⁻¹ run over 60 s using 0.1 cm³ of product at 20°C.

The term "oral care benefit agent" as used herein refers to any composition which has a prophylactic, therapeutic or cosmetic benefit either directly within the oral cavity or which is absorbed via the oral cavity but which has its primary benefits elsewhere.

The term "oral cavity" as used herein refers to the cavity from the lips to the epiglottis. The "hard tissues" comprise tissues such as the teeth and periodontal support and the like, and the "soft tissues" comprise tissues such as the gums, the tongue, the surfaces of the buccal cavity and the like. Within the scope of this application the hard tissues of the oral cavity should also be considered to comprise any devices which are used therein for example dentures, partial dentures, braces and the like.

Active and other ingredients useful herein may be categorised or described herein by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

Herein, "comprising" means that other steps and other ingredients which do not affect the end result can be added. This term encompasses the terms "consisting of" and "consisting essentially of'. The compositions and methods/processes of the present invention can comprise, consist of, and consist essentially of the essential elements and limitations of the invention described herein, as well as any of the additional or optional ingredients, components, steps, or limitations described herein.

### A. Method of Use

The present invention is directed to a method of applying an aqueous gel comprising at least one oral care benefit agent to the oral cavity such that at least 75% of the gingival margin is coated with the gel. The gingival margin is of importance, as it is this area where plaque formation can result in gingivitis, and lead to periodontal disease. Coating at least 75% of the gingival margin will result in similar levels of coating on the teeth and gums. Application to a substantial proportion of the gingival margin is advantageous in the treatment of plaque. However, for stain removal and anti-caries activity it is desirable that the gel be applied so that at least at least 75% of the buccal portion of the hard tissue is coated. Preferably application is such that 100% of the gingival margin and the hard tissues are coated by the gel. The application is such that the gel is applied to the oral cavity before sleeping and is not intentionally removed from the oral cavity by way of rinsing or mechanical brushing or other such like means before sleeping. It has been found that this method is advantageous in combating the degeneration of the oral cavity overnight and reducing morning mouth malodour.

The method comprises the application of the aqueous gel to the oral cavity by the consumer as part of the daily oral hygiene regimen after completing brushing, mouth washing, treatment with dental floss and other such like activities associated with the maintenance of oral hygiene. More preferable is the method wherein the application of the aqueous gel to the oral cavity follows the completion of oral hygiene activities by the consumer, and prior to sleeping. Preferably the gel is maintained on, and releases contained oral care benefit agents onto, the tissues of the oral cavity for an extended period of time not less than 15 minutes, preferably not less than 30 minutes and more preferably not less than 1 hour.

The aqueous gel to be applied according to the current invention preferably has a combination of good stability characteristics when applied to the oral cavity. Preferably, the oral care benefit agents of the gel are released onto the oral tissues from the gel after application, and remain adherent to the oral tissues following dissolution of the carrier gel matrix for a period of time not less than 1 hour, preferably 4 hours, more preferably 6 hours and more preferably still 8 hours. This method of sustained delivery is advantageous as sustained delivery of oral care agents to the oral cavity has previously been achieved using insoluble polymers and vehicles that require mechanical removal following treatment, or solid phase support mechanisms that do not have high consumer aesthetics.

The aqueous gel herein does not form a layer on the oral tissues that is palpable to the consumer upon waking, nor does it require mechanical means of removal.

### B. The Aqueous Gel

### Oral Care Benefit Agents

The gel of the present invention comprises at least one oral care benefit agent. Oral care benefit agents of the present invention may be selected from the group including anti-microbial agents, desensitising agents, teeth whitening actives, anti-stain agents, anti-tartar agents, anti-plaque agents, fluoride ion sources, tooth strengthening agents, nutrients, antioxidants, H-2 antagonists and mixtures thereof. The oral care benefit agent may comprise from about 0.01% to about 15% by weight of the gel. The following is a non exclusive list of oral care benefit agents that may be used in the present invention:

### 1. Teeth Whitening Actives

Teeth whitening actives may be included in the oral care benefit agent of the present invention. The actives suitable for whitening are selected from the group consisting of the peroxides, metal chlorites, perborates, percarbonates, peroxyacids, and combinations thereof. Suitable peroxide compounds include hydrogen peroxide, calcium peroxide, carbamide peroxide, and mixtures thereof. Suitable metal chlorites include calcium chlorite, barium chlorite, magnesium chlorite, lithium chlorite, sodium chlorite, and potassium chlorite. Additional whitening actives may be the hypochlorite salts and chlorine dioxide.

### 2. Anti-tartar agents

Anti-tartar agents known for use in dental care products include pyrophosphates, linear polyphosphates with 4 or more repeat units, polyphosphonates and mixtures thereof. Pyrophosphate ions delivered to the teeth are derived from pyrophosphate salts. The pyrophosphate salts are described in more detail in Kirk & Othmer, *Encyclopedia of Chemical Technology,* Third Edition, Volume 17, Wiley-Interscience Publishers (1982). Agents that may be used in place of or in combination with pyrophosphate salts include such known materials as synthetic anionic polymers including polyacrylates and copolymers of maleic anhydride or acid and methyl vinyl ether, as described, for example, in U.S. Patent 4,627,977, to Gaffar et al.; as well as, e.g., polyamino propoane sulfonic acid (AMPS), zinc citrate trihydrate, linear polyphosphates (e.g., tripolyphosphate; hexametaphosphate), diphosphonates (e.g., ethane-1-hydroxy-1,1-diphosphonate, 1-azacycloheptane-1,1-diphosphonate), polypeptides (such as polyaspartic and polyglutamic acids), and mixtures thereof. Further antitartar agents include polycarboxylates; polyepoxysuccinates; ethylenediaminetetraacetic acid; linear alkyl diphosphonates; linear carboxylic acids; sodium zinc citrate, nitrilotriacetic acid and related compounds.

### 3. Fluoride Ion Source

Fluoride ion sources are well known for use in oral care compositions as anticaries agents. Fluoride ions are contained in a number of oral care compositions for this purpose. A wide variety of fluoride ion-yielding materials can be employed as sources of soluble fluoride in the instant aqueous gels. Examples of suitable fluoride ion-yielding materials include sodium fluoride, stannous fluoride and sodium monofluorophosphate. Preferably the instant aqueous gels provide from about 50 ppm to 10,000 ppm, more preferably from about 100 to 3000 ppm, of fluoride ions in the aqueous solution.

### 4. Antimicrobial Agents

Preferred oral care benefit agents herein are anti-microbial agents. Antimicrobial agents are known to those skilled in the art and include cationic agents, non-cationic agents and metal ion salts. Such agents may include, but are not limited to, 5-chloro-2-(2,4-dichlorophenoxy)-phenol, commonly referred to as triclosan, and described in The Merck Index, 11th ed. (1989), pp. 1529 (entry no. 9573); phthalic acid and its salts, substituted monoperthalic acid and its salts and esters, preferably magnesium monoperoxy phthalate, chlorhexidine (Merck Index, no. 2090), alexidine (Merck Index, no. 222; hexetidine (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; nicin preparations; zinc/stannous ion agents; antibiotics such as augmentin, amoxicillin, tetracycline, doxycycline, minocycline, and metronidazole; and analogs and salts of the above; essential oils including thymol, geraniol, carvacrol, citral, hinokitiol, eucalyptol, catechol (particularly 4-allyl catechol) and mixtures thereof; methyl salicylate; hydrogen peroxide; nanochitosan, metal salts of chlorite and mixtures of all of the above. Preferred antimicrobial agents include cetyl pyridinium chloride and triclosan. Preferably the antimicrobial agent comprises from about 0.05% to about 3%, more preferably from about 0.1% to about 1.5% by weight of the aqueous gel.

### 5. Anti-inflammatory Agents

Anti-inflammatory agents can also be present in the aqueous gel of the present invention. Such agents may include, but are not limited to, non-steroidal anti-inflammatory agents (or NSAIDs) such as ketorolac, flurbiprofen, ibuprofen, naproxen, indomethacin, aspirin, ketoprofen, piroxicam and meclofenamic acid. Use of NSAIDs such as Ketorolac are claimed in U.S. Patent 5,626,838, issued May 6, 1997. Disclosed therein are methods of preventing and, or treating primary and reoccurring squamous cell carcinoma of the oral cavity or oropharynx by topical administration to the oral cavity or oropharynx an effective amount of an NSAID.

### 6. Nutrients

Nutrients may improve the condition of the oral cavity and can be included in the aqueous gels of the present invention. Nutrients include minerals, vitamins, nutritional supplements, and mixtures thereof.

Minerals that can be included with the aqueous gels of the present invention include calcium, phosphorus, fluoride, zinc, manganese, potassium and mixtures thereof. These minerals are disclosed in Drug Facts and Comparisons (loose leaf drug information service), Wolters Kluer Company, St. Louis, Mo., ©1997, pp10-17.

Vitamins can be included with minerals or used separately. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Such vitamins are disclosed in Drug Facts and Comparisons (loose leaf drug information service), Wolters Kluer Company, St. Louis, Mo., © 1997, pp. 3-10.

Nutritional supplements include amino acids, lipotropics, fish oil, protein products, glucose polymers, corn oil, safflower oil, medium chain triglycerides and mixtures thereof, as disclosed in Drug Facts and Comparisons (loose leaf drug information service), Wolters Kluer Company, St. Louis, Mo., ©1997, pp. 54-54e. Amino acids include, but, are not limited to L-Tryptophan, L-Lysine, Methionine, Threonine, Levocarnitine or L- carnitine and mixtures thereof. Lipotropics include, but are not limited to choline, inositol, betaine, linoleic acid, linolenic acid, and mixtures thereof. Fish oil contains large amounts of Omega-3 (N-3) polyunsaturated fatty acids, eicosapentaenoic acid and docosahexaenoic acid.

### 7. Enzymes

An individual or combination of several compatible enzymes can be included in the aqueous gel of the present invention. Enzymes are biological catalysts of chemical reactions in living systems. Enzymes combine with the substrates on which they act forming an intermediate enzyme-substrate complex. This complex is then converted to a reaction product and a liberated enzyme which continues its specific enzymatic function.

Enzymes provide several benefits when used in the oral cavity. Proteases break down salivary proteins which are absorbed onto the tooth surface and form the pellicle; the first layer of plaque. Proteases along with lipases destroy bacteria by lysing proteins and lipids which form the structural component of bacterial cell walls and membranes. Dextranases break down the organic skeletal structure produced by bacteria that forms a matrix for bacterial adhesion. Proteases and amylases, not only present plaque formation, but also prevent the development of calculus by breaking-up the carbohydrate-protein complex that binds calcium, preventing mineralization.

Enzymes useful in the present invention include any of the commercially available proteases, glucanohydrolases, endoglycosidases, amylases, mutanases, lipases and mucinases or compatible mixtures thereof. Preferred are the proteases, dextranases, endoglycosidases and mutanases, most preferred being papain, endoglycosidase or a mixture of dextranase and mutanase.

### 8. Antioxidants

Antioxidants are generally recognized as useful in aqueous gels such as those of the present invention. Antioxidants are disclosed in texts such as Cadenas and Packer, The Handbook of Antioxidants, © 1996 by Marcel Dekker, Inc. Antioxidants that may be included in the aqueous gel or substance of the present invention include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

### 9. H-2 Antagonists

Histamine-2 (H-2 or H?) receptor antagonist compounds (H-2 antagonists) may be used in the aqueous gel of the present invention. H-2 antagonists are compounds that block H-2 receptors, but do not have meaningful activity in blocking histamine-1 (H-1 or H?) receptors. H-2 antagonists stimulate the contraction of smooth muscle from various organs, such as the gut and bronchi; this effect can be suppressed by low concentrations of mepyramine- a typical antihistaminic drug. The pharmacological receptors involved in these mepyramine-sensitive histamine responses have been defined as H-1 receptors (Ash, A.S.F. & H.O. Schild, Brit. J. Pharmacol Chemother., Vol. 27 (1966), p. 427). The H-2 antagonists useful in the aqueous gels are those that block the receptors involved in mepyramine-insensitive, non-H-1 (H-2), histamine responses, and do not block the receptors involved in mepyramine-sensitive histamine responses.

H-2 antagonists meeting the above criteria include cimetidine, etintidine, ranitidine, ICIA-5165, tiotidine, ORF-17578, lupitidine, donetidine, famotidine, roxatidine, pifatidine, lamtidine, BL-6548, BMY-25271, zaltidine, nizatidine, mifentidine, BMY-52368, SKF-94482, BL-6341A, ICI-162846, ramixotidine, Wy-45727, SR-58042, BMY-25405, loxtidine, DA-4634, bisfentidine, sufotidine, ebrotidine, HE-30-256, D-16637, FRG-8813, FRG-8701, impromidine, L-643728, and HB-408. 4, as disclosed in US 5,294,433 and 5,364,616.

### Thickeners

The method of the present invention comprises application of a gel. The gel is a high viscosity matrix formed from thickeners known in the art which are safe for oral use and do not react with or inactivate the oral care benefit agents incorporated into them. Furthermore, the gel formed with these thickeners may provide sufficient adhesive attachment to the teeth or mucosa to keep them coated for a period of not less than 15 minutes.

The amount of thickener required to form the gel is such that the viscosity of the gel is greater than about 10 Pa.s at a shear rate of 0.1 s⁻¹. This development produces a gel that, when placed on an applicator or finger, does not run off or prove too runny to use effectively. The amount of thickener is such that the viscosity is from about 0.1 Pa.s to about 300 Pa.s, preferably from about 30 Pa.s to about 200 Pa.s, and more preferably from about 80 Pa.s to about 120 Pa.s at a shear rate of 1 s⁻¹. This is advantageous to create a gel with good aesthetics and consumer compliance, and enable the gel to be spread effectively across the oral tissues, yet remain substantive on those tissues following application.

Suitable thickening agents useful in the present invention include polysaccharide thickeners, clays, cross-linked poly-acrylates, co-polymers, polyethylene glycols and derivatives, protein thickeners and mixtures thereof. Preferred levels of thickener to form the gel are from about 0.1% to about 15%, preferably from about 0.5% to about 10%, more preferably from about 2% to about 5%, by weight.

Polysaccharide thickeners useful in the present invention include hydroxypropylmethylcellulose (HPMC), hydroxypropylcellulose (HPC), hydroxyethylcellulose (HEC), carboxymethylcellulose (CMC, cellulose gum), methylcellulose, cetylhydroxyethylcellulose, methylhydroxyethylcellulose, microcrystalline cellulose, hydroxyethylethylcellulose, methylhydroxypropylcellulose, carboxymethylhydroxyethylcellulose, xanthan gum, sclerotium gum, carboxymethyl hydroxypropyl guar, guar gum, glyceryl alginate, guar (cyanopsis tetragonoloba) gum, guar hydroxypropyltrimonium chloride, gum arabic/gum acacia, hydroxypropyl guar, karaya (sterculia urens) gum, gellan gum, agar, carrageenan (kappa, iota, lambda), pectin, locust bean (ceratonia siliqua) gum, carboxymethyl chitosan, hydroxyethyl chitosan, carboxymethyl dextran, corn (zea mays) starch, dextrin, potassium alginate, potato starch modified, propylene glycol alginate, sodium carboxymethyl betaglucan, sodium carboxymethyl dextran, sodium carboxymethyl starch, sodium hydroxypropyl starch phosphate, maltodextrin, algin/alginic acid, and mixtures thereof.

Clays useful in the present invention include sodium magnesium silicate, lithium magnesium silicate, lithium magnesium sodium silicate, sodium magnesium fluorolithosilicate, bentonite, montmorillonite clay and mixtures thereof.

Cross-linked polyacrylates useful in the present invention include sodium acrylate/vinyl alcohol copolymer, acrylate/c10-30 alkyl acrylate crosspolymer, acrylates/ceteth-20 itaconate copolymer, acrylates/ceteth-20 methacrylate copolymer, acrylates/steareth-50 acrylate copolymer, acrylates/steareth-20 itaconate copolymer, acrylates/steareth-20 methacrylate copolymer, carbomer, glycerin/glyceryl polyacrylate and mixtures thereof.

Synthetic copolymers useful in the present invention include Poloxamer 407, PVM/MA co-polymer, (commercially available under the trade name "Gantrez"), PVP (poly(vinylpyrrolidone)), polyacrylamideomethylpropane sulfonic acid and mixtures thereof.

Polyethylene glycols useful in the present invention include PEG -2M, PEG-5M, PEG-7M, PEG-9M, PEG-14M, PEG-20M, PEG-23M, PEG-25M, PEG-45M, PEG-90M, PEG-115M, PEG-160M, PEG-crosspolymer, PEG-140 glyceryl tristearate and mixtures thereof.

Preferred thickeners for use in the present invention are the polysaccharide thickeners and the co-polymers. More preferred are the water-soluble cellulosic and acrylic thickeners. Most preferred is HPMC. The aqueous gel may comprise from about 2.1% to 4.9% HPMC, preferably from 2.5% to 4.7% and more preferably from 2.8% to 4.3% by weight. It has been found that some oral care benefit agents react with thickeners to modify the visocisty of the gel synergistically. More specifically, it has been found that combinations of quaternary anti-microbials with cellulosic thickeners thickens the gel more effectively than when cellulosic thickeners are used on their own. Preferred are the cellulosic derivatives such as e.g. HPMC and HEC in combination with the cationic surfactant antimicrobials. Preferred is the combination of greater than 0.02% cetylpyridinium chloride (CPC) and from about 2.1% to about 4.9% HPMC. Incorporation of CPC with HPMC at these levels results in a marked increase in the viscosity of the gel when compared with HPMC alone. Without wishing to be bound by theory, it is thought that when the levels of CPC reach the critical micelle concentration, which in the literature is reported to be about 0.017% by weight, the CPC interacts with thickening agents such as HPMC and HEC to thicken the gel significantly more than HPMC or HEC alone.

The water present in the gel should preferably be deionized and free of organic impurities. Water typically comprises from about 0.1% to 95%, preferably from about 5% to about 90%, and most preferably from about 10% to about 80%, by weight of the gel. This amount of water includes the free water that is added plus that amount that is introduced with other materials.

The aqueous gel for use in the present invention may optionally comprise xylitol. Xylitol is a polyol that may be added to provide sweetening and flavouring. Furthermore, xylitol may have some positive benefits as an anti-caries agent. The aqueous gel may comprise from about 0.1% to about 15% xylitol, preferably from about 1% to 10%, and more preferably from about 2% to 8% xylitol.

A pH adjusting agent may also be added to optimize the storage stability of the gel and to make the substance safe for oral tissue. These pH adjusting agents, or buffers, can be any material which is suitable to adjust the pH of the aqueous gel. Suitable materials include sodium bicarbonate, sodium phosphate, sodium hydroxide, ammonium hydroxide, sodium stannate, triethanolamine, citric acid, hydrochloric acid, sodium citrate, and combinations thereof. The pH adjusting agents are generally added in sufficient amounts so as to adjust the pH of the gel to about 4.5 to about 11, preferably from about 5 to about 9, and more preferably from about 5 to about 8. pH adjusting agents are generally present in an amount of from about 0.01% to about 15% and preferably from about 0.05% to about 5%, by weight.

An additional carrier material may also be added to the aqueous gel. Carrier materials can be humectants. Suitable humectants include glycerin, sorbitol, polyethylene glycol, propylene glycol, and other edible polyhydric alcohols. Humectants are generally present in an amount of from about 10% to about 50% and preferably from about 15% to about 40%, by weight of the aqueous gel. In addition to the above materials the gel of the present invention may comprise a number of other components. Additional components include, but are not limited to, flavoring agents, sweetening agents, opacifiers, coloring agents, emulsifiers and chelants such as ethylenediaminetetraacetic acid. These additional ingredients can also be used in place of the compounds disclosed above. Flavours, sweetners, colours and sensates may comprise from about 0.1% to about 10% by weight of the gel for use in the current invention.

In addition, the aqueous gel of the present invention preferably comprises not more than about 18% C₁-C₆ monohydric alcohols. Higher alcohol levels in a gel intended for overnight use are potentially deleterious. However, it is known to those skilled in the art that polyhydric alcohols disclosed above are useful as humectants in gels. Preferably, the aqueous gel contains less than 10% monohydric alcohols, more preferably less than 5%, and more preferably still contains no monohydric alcohols. These levels are desirable to maintain safety and gel aesthetics.

Similarly, the aqueous gel preferably comprises less than 5% abrasives. Abrasives, whilst useful in dentifrices, are not desirable in the current invention. Preferably the gel comprises less than 4% abrasives, more preferably less than 2% abrasives and more preferably still comprises no abrasives. The applicant has found that low levels of abrasives are desirable to maintain consumer compliance and good gel aesthetics.

The aqueous gel of the present invention may comprise moderate levels of silicones. Silicones may be desirable to aid the modification of the rheology and substantivity. However, high levels of silicones are undesirable as some consumers would prefer the gel not to be as substantive as gels containing higher levels of silicones. Gels with moderate levels of silicones are desirable as they provide improved mouth feel and sensate delivery. Aqeous gels for use in the present invention comprise less than 10%, preferably from about 0.05% to about 9%, more preferably from about 0.1% to about 8%, by weight, of a silicone. Suitable silicones for use in the present invention include those disclosed in WO 01/01940. Preferred silicones include silicone resins, silicone gums and silicone fluids having a viscosity, at 25°C, of from about 1x10⁻⁶ m²/s to about 1x10⁻³ m²/s. More preferred are the silicone fluids. More preferred still are the polysiloxane fluids include linear polysiloxane polymers such as the linear dimethicones having a molecular weight of at least 4000 and where R is a methyl substituent, and other low viscosity analogues of the polysiloxane materials. Also preferred are the alkyl and alkoxy substituted dimethicone polyols as disclosed in WO 96/33693.

In an embodiment, the present invention may comprise a kit for the application of overnight oral gel, comprising an oral care gel, an applicator and method of use instructions directed towards application and overnight use of the gel therein.

The aspects and embodiments of the present invention set forth in this document have many advantages. For example, they can provide increased efficacy of delivery of oral care benefit agents that provide for better oral hygiene. Similarly, overnight treatment of the oral cavity may result in a reduction of "morning mouth" experienced by the consumer. Furthermore, overnight application of oral care benefit agents may result in effective reduction in the degeneration of the oral cavity accelerated by the conditions imparted by sleeping. Various embodiments of the present invention address the need for better consumer aesthetics and appeal of intensive oral treatments combined with increased ease of application.

The following examples further describe and demonstrate the preferred embodiments within the scope of the present invention. The examples are given solely for the purpose of illustration, and are not to be construed as limitations of the present invention since many variations thereof are possible without departing from its scope.

The gels of examples I to VIII (table A) were produced and found to be suitable for use in the present invention. The gel of example IX was found to not be suitable according to the present invention.

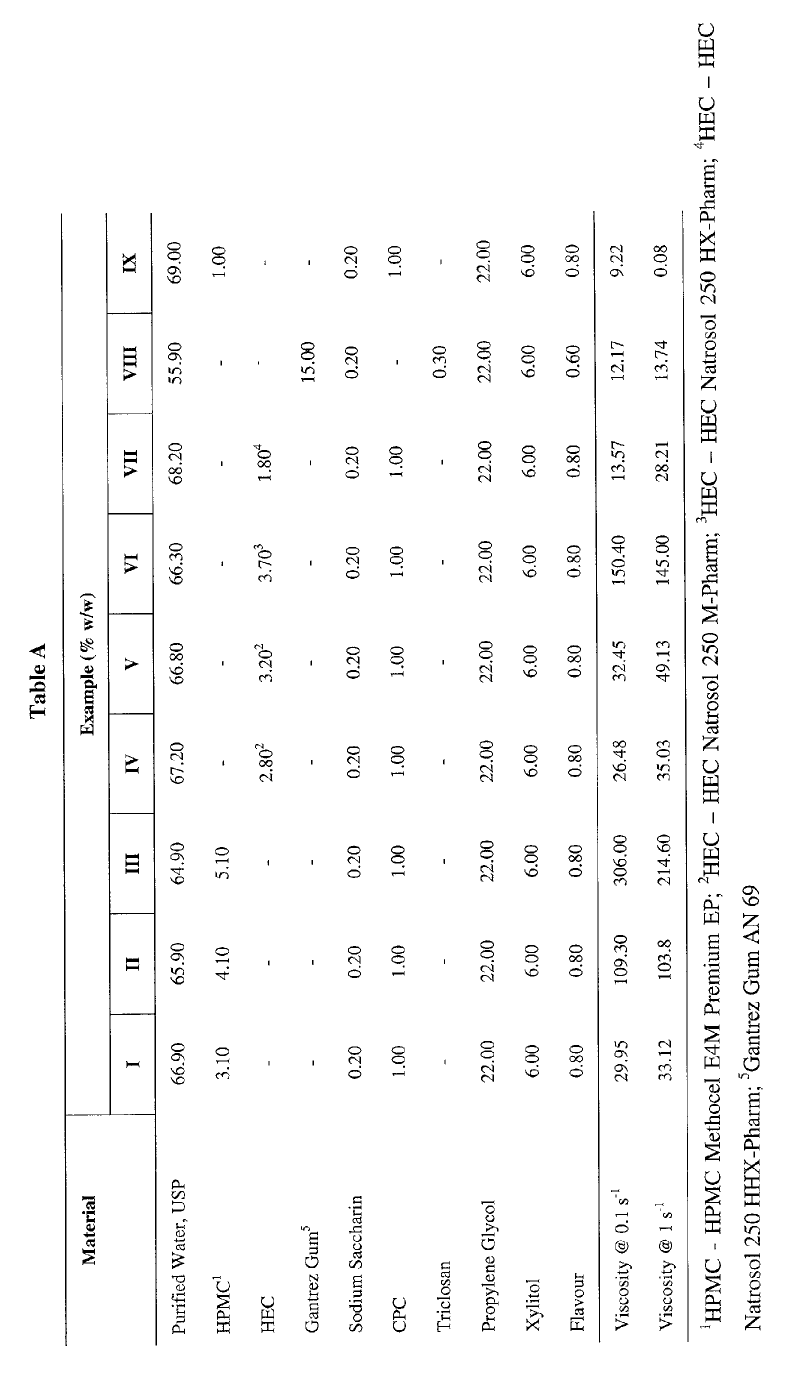

## Claims

1. A method for treating the oral cavity comprising the application of an aqueous gel to the oral cavity such that at least 75% of the gingival margin or at least at least 75% of the buccal portion of the hard tissue is coated with the gel, application occurring as part of the daily oral care routine shortly before retiring, and the gel remaining in contact with the oral tissues while sleeping, the gel comprising;
a) an oral care benefit agent;
b) a thickener;
c) less than 5% abrasive;
d) less than 18% C₁-C₆ monohydric alcohols;
e) less than 10% silicone; and
the gel further having a viscosity of greater than about 10 Pa.s at a shear rate of 0.1 s⁻¹ and from about 0.1 Pa.s to about 300 Pa.s. at a shear rate of 1 s⁻¹.

2. The method of Claim 1 wherein the oral care benefit agent is selected from the group consisting of anti-microbial agents, desensitising agents, anti-stain agents, anti-tartar agents, anti-plaque agents, fluoride ion sources, tooth strengthening agents, nutritients, antioxidants, H-2 antagonists and mixtures thereof and comprises between 0.01% and 5% by weight.

3. The method according to claims 1 and 2 wherein the oral care benefit agent is an anti-microbial.

4. The method according to claims 1 to 3 wherein the oral care benefit agent is cetylpyridinium chloride.

5. The method according to claims 1 to 3 wherein the oral care benefit agent is triclosan.

6. The method according to any of the preceding claims wherein the thickening agent is selected from the list including polysaccharide thickeners, clays, cross-linked poly-acrylates, co polymers, polyethylene glycols and derivatives, protein thickeners or a mixture thereof, and comprises from about 0.1% to 15% by weight of the gel.

7. The method according to any of the preceding claims wherein the aqueous gel comprises polysaccharide thickeners, preferably hydroxypropylmethylcellulose.

8. The method according to any of the preceding claims wherein the aqueous gel comprises synthetic co-polymers, preferably PMV/MA co-polymer.

9. The method according to any of the preceding claims wherein the gel has a viscosity of from about 30 Pa.s to about 200 Pa.s, preferably from about 80 Pa.s to about 120 Pa.s at a shear rate of 1 s⁻¹.

10. The method according to any of the preceding claims wherein the gel comprises from 10% to 50% of a humectant chosen from the group consisting of sugar alcohols, dihydric alcohols, polyhydric alcohols and mixtures thereof.

11. The method according to any of the preceding claims wherein the gel comprises from about 0.1% to 15% xylitol.

12. An oral composition for use according to claim 1 comprising;
a) From about 0.1% to about 1.5% by weight of an anti-microbial;
b) From about 1% to about 15% thickener;
c) Less than 5% abrasive;
d) Less than 18% C₁-C₆ monohydric alcohols;
e) Less than 10% silicone; and
the oral composition being **characterised in that** it has a viscosity of greater than about 10 Pa.s at a shear rate of 0.1 s⁻¹ and from about 0.1 Pa.s to about 300 Pa.s. at a shear rate of 1 s⁻¹.

13. The oral composition according to claim 12 wherein the anti-microbial is cetylpyridinium chloride.

14. The oral composition according to claim 12 wherein the anti-microbial is triclosan.

15. The oral composition according to claims 12 to 14 comprising hydroxypropylmethylcellulose.

16. The oral composition according to claims 12 to 15 wherein the level of hydroxypropylmethylcellulose is from about 2.1% to about 4.9%, preferably from about 2.5% to about 4.7%, and more preferably from about 2.8% to about 4.3%.

17. The oral composition according to claims 12 to 14 comprising synthetic co-polymers, preferably PMV/MA compolymer.

18. The oral composition according to claims 12 to 16 comprising cetylpyridinium chloride and hydroxypropylmethylcellulose.

19. The oral composition according to claims 12 to 18 having a viscosity of from about 30 Pa.s to about 200 Pa.s, preferably from about 80 Pa.s to about 120 Pa.s at a shear rate of 1 s⁻¹.

20. A kit for treatment of the oral cavity according to claim 1 comprising an oral care gel, an applicator and method of use instructions indicating overnight application to the oral cavity.
